# EUROPEAN PATENT APPLICATION

(11) **EP 4 834 386 A1**
(43) Date of publication of application: **04.11.2026**
(21) Application number: 25754621.8
(22) Date of filing: 13.02.2025
(51) Int. Cl.: A61M 1/16, A61M 1/36

(54) **BLOOD PURIFICATION DEVICE**

(30) Priority: 14.02.2024 JP 2024020716; 14.02.2024 JP 2024020717
(71) Applicant: Nikkiso Company Limited, Tokyo 150-6022 (JP)
(72) Inventor: KATAYAMA Yuki, Higashimurayama-shi, Tokyo 189-8520 (JP)
(74) Representative: von Hirschhausen, Helge
(86) International application number: PCT/JP2025/004809
(87) International publication number: WO 2025/173750

(57) **Abstract**

A blood purification apparatus that allows an operator such as a medical worker to change the start point or the end point of a local characteristic variation as desired and to simulate the calculation of the rate of recirculating blood includes a characteristic-variation-imparter capable of imparting a local concentration variation to a fluctuating concentration of the blood extracorporeally circulating through a blood circuit (1), the local concentration variation having a unique peak; a first detector (5a) and a second detector (5b) each being configured to detect the local concentration variation imparted by the characteristic-variation-imparter; and a recirculation-detector (17) configured to detect any recirculating blood using the local concentration variation β detected by the first detector (5a) and the local concentration variation α detected by the second detector (5b), and to calculate a rate of the recirculating blood. The blood purification apparatus further includes a changing operation unit (19) configured to change identified start points (R1 and W1) or end points (R2 and W2) as desired.

## Description

### Technical Field

The present invention relates to a blood purification apparatus configured to detect any recirculating blood, which is blood reintroduced into an arterial blood circuit after being returned to a patient from a venous blood circuit.

### Background Art

In typical blood purification treatment such as hemodialysis treatment, while a patient's blood is caused to extracorporeally circulate through a blood circuit, the blood is purified by a dialyzer. However, for example, in a case where extracorporeal circulation is implemented through the patient's shunt (a site where an artery and a vein are connected to each other by a surgical operation) that is punctured with an arterial puncture needle and a venous puncture needle, recirculating blood may occur. Recirculating blood is the purified blood returned into the patient's body through the venous puncture needle but reintroduced into the blood circuit through the arterial puncture needle without flowing through the patient's organs and so forth. The occurrence of such recirculating blood means that the purified blood further undergoes extracorporeal circulation, which correspondingly reduces the amount of extracorporeal circulation of the blood that needs to be purified, leading to a problem of reduction in the blood purification efficiency.

To detect such recirculating blood, a dialysis apparatus proposed in PTL 1, for example, is capable of detecting any blood recirculation by activating an ultrafiltration pump such that a unique peak serving as a marker is imparted to the concentration of blood that fluctuates while the blood is under extracorporeal circulation. In the dialysis apparatus disclosed in PTL 1, a sensor configured to detect blood concentration (a sensor configured to detect hemoglobin concentration) is provided to the arterial blood circuit. With the detection of the unique peak by the sensor, blood recirculation that may occur during dialysis treatment can be detected.

### Citation List

### Patent Literature

PTL 1: JP2000-502940 (a Published Japanese Translation of a PCT Application)

### Summary of Invention

### Technical Problem

However, in terms of calculating the rate of recirculating blood for obtaining the recirculation rate and/or the flow rate at the patient's shunt, the known blood purification apparatus has the following problem.

In the technique of imparting a local concentration variation, the result of detection of the local concentration variation may vary with the patient's condition, the treatment environment, and so forth. Therefore, to calculate the rate of recirculating blood with high accuracy, the start point and the end point of the local concentration variation need to be identified accurately. If such a local concentration variation detected by a detector is used in calculating the rate of recirculating blood, since the start point and/or the end point of the local concentration variation taken as grounds for the calculation may be unclear, the medical worker may have a desire to freely change the start point and/or the end point. The above problem also applies to a case where the rate of recirculating blood is calculated using fluctuations in another characteristic of blood such as blood temperature, instead of blood concentration. Note that the characteristic of blood is not limited to blood concentration and encompasses all blood characteristics including blood temperature.

The present invention has been conceived in view of the above circumstances and provides a blood purification apparatus that allows an operator such as a medical worker to change the start point or the end point of the local characteristic variation as desired and to simulate the calculation of the rate of recirculating blood.

### Solution to Problem

According to an embodiment of the present invention, there is provided a blood purification apparatus configured to purify a patient's blood by causing the blood to extracorporeally circulate through a blood circuit including an arterial blood circuit and a venous blood circuit. The blood purification apparatus includes a characteristic-variation-imparter capable of imparting a local characteristic variation to a fluctuating characteristic of the blood extracorporeally circulating through the blood circuit, the local characteristic variation having a unique peak; a first detector and a second detector provided to the arterial blood circuit and the venous blood circuit, respectively, and each being configured to detect the local characteristic variation imparted by the characteristic-variation-imparter; and a recirculation-detector configured to detect any recirculating blood using the local characteristic variation detected by the first detector and the local characteristic variation detected by the second detector, and to calculate a rate of the recirculating blood, the recirculating blood being blood reintroduced into the arterial blood circuit after being returned to the patient from the venous blood circuit. The recirculation-detector is configured to identify start points or end points of the local characteristic variations detected by the first detector and the second detector. The blood purification apparatus further includes a changing operation unit configured to change the identified start points and end points as desired.

### Advantageous Effects of Invention

According to the present invention, the recirculation-detector is configured to identify the start points or the end points of the local characteristic variations detected by the first detector and the second detector. Furthermore, the blood purification apparatus includes the changing operation unit configured to change the identified start points or end points as desired. Such a configuration enables an operator such as a medical worker to determine the start points or the end points of the local characteristic variations as desired, and to simulate the calculation of the rate of recirculating blood.

### Brief Description of Drawings

[Fig. 1] Fig. 1 is a diagram illustrating a blood purification apparatus according to an embodiment of the present invention.
[Fig. 2] Fig. 2 is a block diagram illustrating a main configuration of the blood purification apparatus.
[Fig. 3] Fig. 3 is a block diagram illustrating a recirculation-detector included in the blood purification apparatus.
[Fig. 4] Fig. 4 is a graph illustrating local concentration variations detected by a second detector and a first detector included in the blood purification apparatus.
[Fig. 5] Fig. 5 is a flow chart illustrating a control process that is performed by a controller included in the blood purification apparatus.
[Fig. 6] Fig. 6 is a flow chart illustrating a control process that is performed by the controller included in the blood purification apparatus and that is relevant to an operation of a changing operation unit.
[Fig. 7] Fig. 7 is a schematic diagram illustrating what is displayed on a display included in the blood purification apparatus.

### Description of Embodiments

An embodiment of the present invention will now be described specifically with reference to the drawings.

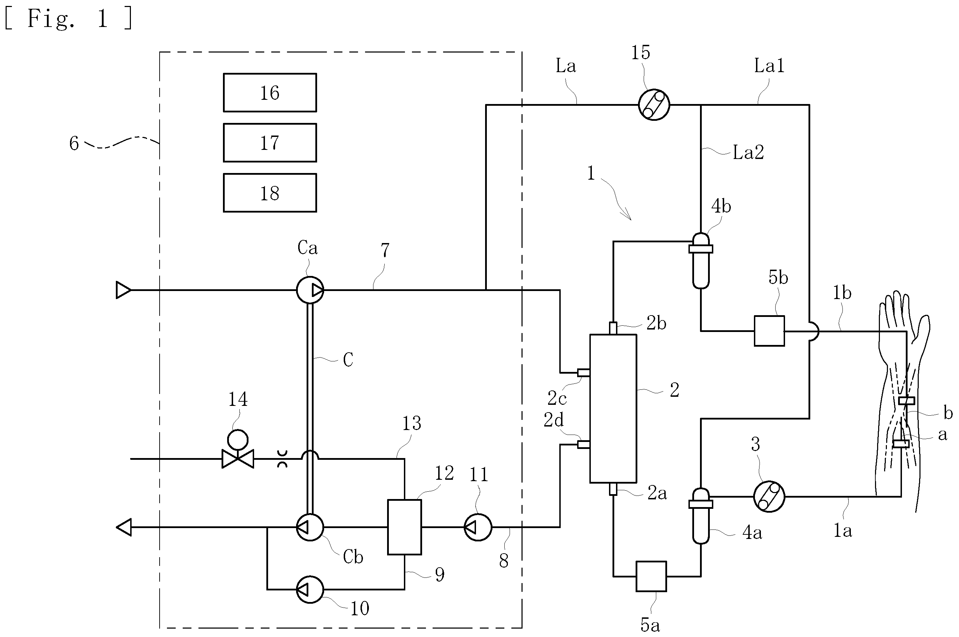

A blood purification apparatus according to the present embodiment is intended to purify a patient's blood while causing the blood to extracorporeally circulate, and is applied to a hemodialysis apparatus intended for hemodialysis treatment. As illustrated in Fig. 1, the hemodialysis apparatus includes a dialysis device 6, to which a blood circuit 1 provided with a dialyzer 2 serving as a blood purification unit is attachable. The dialysis device 6 is configured to supply dialysate to the dialyzer 2 and simultaneously perform ultrafiltration. The hemodialysis apparatus further includes a controller 16, a recirculation-detector 17, a display 18, and a changing operation unit 19, which are provided in the dialysis device 6.

As illustrated in the drawing, the blood circuit 1 is mainly constituted by an arterial blood circuit 1a and a venous blood circuit 1b, which are made of flexible tubes. The dialyzer 2 is connected between the arterial blood circuit 1a and the venous blood circuit 1b. The arterial blood circuit 1a is provided with an arterial puncture needle a at the distal end thereof. The arterial blood circuit 1a is further provided with a peristaltic blood pump 3, an air-trap chamber 4a for bubble removal, and a first detector 5a at respective halfway positions thereof. The venous blood circuit 1b is provided with a venous puncture needle b at the distal end thereof. The venous blood circuit 1b is further provided with a second detector 5b and an air-trap chamber 4b for bubble removal at respective halfway positions thereof.

When the blood pump 3 is activated with the patient being punctured with the arterial puncture needle a and the venous puncture needle b, the patient's blood flows through the arterial blood circuit 1a while being subjected to bubble removal at the air-trap chamber 4a and reaches the dialyzer 2, where the blood is purified. Then, the blood flows through the venous blood circuit 1b while being subjected to bubble removal at the air-trap chamber 4b and returns into the patient's body. Thus, the patient's blood is purified at the dialyzer 2 while being caused to extracorporeally circulate through the blood circuit 1.

The dialyzer 2 has a housing provided with a blood introduction port 2a, a blood delivery port 2b, a dialysate introduction port 2c, and a dialysate delivery port 2d. The proximal end of the arterial blood circuit 1a is connected to the blood introduction port 2a. The proximal end of the venous blood circuit 1b is connected to the blood delivery port 2b. The dialysate introduction port 2c and the dialysate delivery port 2d are connected to a dialysate introduction line 7 and a dialysate drain line 8, respectively. The dialysate introduction line 7 and the dialysate drain line 8 extend from the dialysis device 6.

The dialyzer 2 houses a plurality of hollow fibers. Spaces inside the respective hollow fibers serve as blood flow routes. Spaces between the inner peripheral surface of the housing and the outer peripheral surfaces of the hollow fibers serve as dialysate flow routes. The hollow fibers each have a number of microscopic holes (pores) extending therethrough from the outer peripheral surface to the inner peripheral surface, thereby forming hollow fiber membranes. Impurities and the like contained in the blood are allowed to permeate through the hollow fiber membranes into the dialysate.

The dialysis device 6 includes a duplex pump C, a bypass line 9 connected to the dialysate drain line 8 while bypassing a discharge side Cb of the duplex pump C, an ultrafiltration pump 10 connected to the bypass line 9, a pressurizing pump 11 configured to cause the dialysate to flow from the dialyzer 2 toward the discharge side Cb of the duplex pump C, a bubble isolation chamber 12, an atmosphere release line 13, and an electromagnetic valve 14.

The duplex pump C is provided astride the dialysate introduction line 7 and the dialysate drain line 8. The duplex pump C is intended to introduce the dialysate from the dialysate introduction line 7 into the dialyzer 2 and to cause the dialysate introduced into the dialyzer 2 to be drained through the dialysate drain line 8. Specifically, the duplex pump C is a metering pump, with the flow rates at a supply side Ca and the discharge side Cb thereof being substantially equal to each other. A dialysate flow route extending from the supply side Ca to the discharge side Cb (specifically, a flow route in the dialysate introduction line 7 that extends on the downstream side relative to the duplex pump C, a flow route in the dialysate drain line 8 that extends on the upstream side relative to the duplex pump C, and the dialysate flow routes in the dialyzer 2) forms a closed flow route (a flow route that is kept hermetic) when the electromagnetic valve 14 is closed.

The pressurizing pump 11 is connected to a position of the dialysate drain line 8 between the dialyzer 2 and the duplex pump C. The pressurizing pump 11 is intended to cause the dialysate to flow from the dialyzer 2 toward the duplex pump C and is a non-positive-displacement pump (a pressure-controlled type) such as a centrifugal pump. The ultrafiltration pump 10, to be described below, is also a non-positive-displacement pump (a pressure-controlled type) such as a centrifugal pump as with the pressurizing pump 11.

The dialysate introduction line 7 is connected at one end thereof to the dialysate introduction port 2c of the dialyzer 2 and at the other end thereof to a dialysate supply device (not illustrated) configured to prepare a dialysate at a predetermined concentration. The dialysate drain line 8 is connected at one end thereof to the dialysate delivery port 2d of the dialyzer 2 and at the other end thereof to draining means (not illustrated). After the dialysate supplied from the dialysate supply device flows through the dialysate introduction line 7 and reaches the dialyzer 2, the dialysate flows through the dialysate drain line 8 and the bypass line 9 and is delivered to the draining means.

The ultrafiltration pump 10 is intended to remove water from the patient's blood flowing through the dialyzer 2. Specifically, when the ultrafiltration pump 10 is activated, the amount of fluid drained through the dialysate drain line 8 becomes greater than the amount of dialysate introduced through the dialysate introduction line 7, because the duplex pump C is of a metering type. Accordingly, the excess amount of water is removed from the blood.

The bubble isolation chamber 12 is a so-called degassing chamber and has a predetermined capacity. The bubble isolation chamber 12 is connected to a position of the dialysate drain line 8 between the pressurizing pump 11 and the duplex pump C, and is capable of trapping bubbles in the dialysate. The bypass line 9 described above and the atmosphere release line 13 extend from the bubble isolation chamber 12. The distal end of the atmosphere release line 13 is open to the atmosphere. The electromagnetic valve 14, serving as valve means, is connected to a halfway position of the atmosphere release line 13.

The electromagnetic valve 14 is openable and closable to open or close the atmosphere release line 13. When the electromagnetic valve 14 is open, the bubble isolation chamber 12 communicates with outside air. When the electromagnetic valve 14 is closed, the bubble isolation chamber 12 is closed to outside air. When the atmosphere release line 13 is opened by operating the electromagnetic valve 14 before or after dialysis treatment, bubbles trapped in the bubble isolation chamber 12 is released to the atmosphere.

The dialysate introduction line 7 according to the present embodiment is provided with a supply line La. The supply line La allows dialysate serving as a substitution fluid to be supplied to the blood circuit 1. The proximal end of the supply line La is connected to a portion of the dialysate introduction line 7 between the supply side Ca of the duplex pump C and the dialysate introduction port 2c of the dialyzer 2. The distal end of the supply line La branches into a pre-substitution supply line La1 and a post-substitution supply line La2, which are connected to the air-trap chamber 4a of the arterial blood circuit 1a and the air-trap chamber 4b of the venous blood circuit 1b, respectively.

The supply line La is provided with a substitution pump 15, which is a peristaltic pump as with the blood pump 3. When the substitution pump 15 is activated during the process of blood purification treatment, the dialysate in the dialysate introduction line 7 is supplied to the arterial blood circuit 1a through the pre-substitution supply line La1 so as to enable pre-substitution, and is also supplied to the venous blood circuit 1b through the post-substitution supply line La2 so as to enable post-substitution.

The atmosphere release line 13 and the electromagnetic valve 14 according to the present embodiment constitute a characteristic-variation-imparter according to the present invention. When the atmosphere release line 13 is opened by operating the electromagnetic valve 14, the blood flowing through the dialyzer 2 is concentrated abruptly and briefly, whereby a local concentration variation (characteristic variation) having a unique peak can be imparted. Specifically, during dialysis treatment, when the atmosphere release line 13 in the closed state is opened by operating the electromagnetic valve 14, the outlet pressure of the pressurizing pump 11 becomes substantially equal to the atmospheric pressure. Accordingly, a high negative pressure is instantaneously generated on the upstream side relative to the pressurizing pump 11. Consequently, the blood flowing through the dialyzer 2 (blood flow route) is subjected to abrupt and brief ultrafiltration (hemoconcentration).

Thus, the blood is subjected to ultrafiltration by a far greater amount and more briefly than with the ultrafiltration pressure that is generated by activating the ultrafiltration pump 10, and a unique peak is imparted to the fluctuating blood concentration (hematocrit value). The opening of the atmosphere release line 13 with the operation of the electromagnetic valve 14 is conducted briefly (in the present embodiment, any duration of 10 seconds or shorter may be set for the opening of the atmosphere release line 13, and satisfactory measurement accuracy is achieved even in one second). Soon after that, the electromagnetic valve 14 is operated to close the atmosphere release line 13.

It is preferable that the duration for opening the atmosphere release line 13 with the operation of the electromagnetic valve 14 be automatically optimized using blood concentration information (information on the patient's blood concentration) and/or pressure information (such as venous pressure and/or dialysate pressure). In the present invention, the phrase "abrupt and brief" refers to an extent and a duration that make the imparted pulse recognizable even after the blood circulates through the circuit. The term "unique" refers to being distinguishable from fluctuation patterns caused by any other factors such as fluctuations in the pump and a movement of the patient's body.

The first detector 5a and the second detector 5b are provided to the arterial blood circuit 1a and the venous blood circuit 1b, respectively, so as to detect the concentration (hematocrit value, specifically) of the blood flowing through the respective flow routes. For example, the first detector 5a and the second detector 5b are each a hematocrit sensor including a light-emitting device such as an LED and a light-receiving device such as a photodiode. The light-emitting device emits light toward the blood, and the light-receiving device receives the light transmitted through or reflected by the blood, whereby the hematocrit value representing the patient's blood concentration is detected.

Specifically, the first detector 5a and the second detector 5b each calculate the hematocrit value representing blood concentration from an electric signal that is output from the light-receiving device. Blood components such as red blood cells and plasma each have an inherent absorption characteristic. Using such a characteristic, the amount of red blood cells required for measuring the hematocrit value is electrooptically quantified. Thus, the hematocrit value is calculable. Near infrared rays emitted from the light-emitting device are incident on the blood and undergo absorption and scattering before being received by the light-receiving device. The rate of light absorption and scattering is analyzed from the intensity of the received rays. Thus, the hematocrit value is calculated.

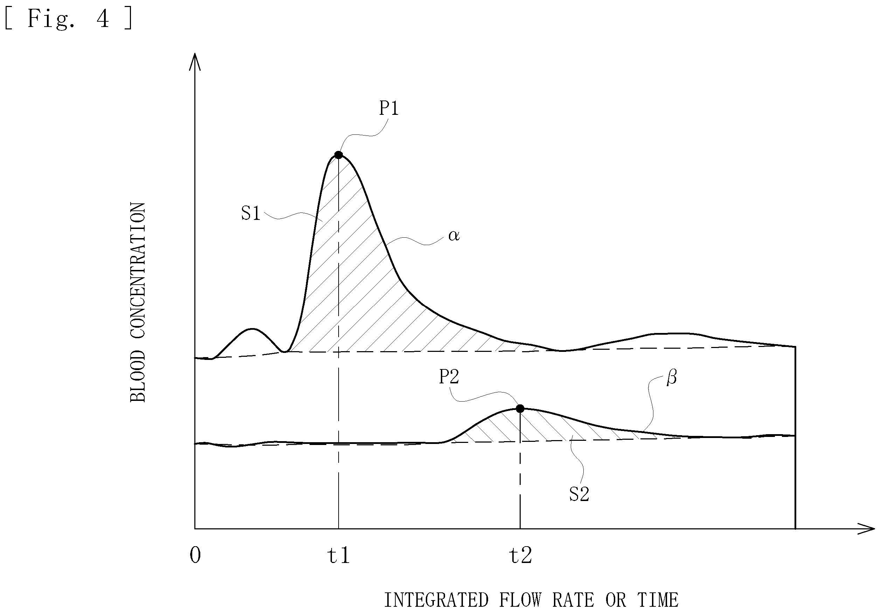

The first detector 5a configured as above is provided to the arterial blood circuit 1a and therefore detects the hematocrit value of the blood collected from the patient through the arterial puncture needle a during dialysis treatment. The second detector 5b is provided to the venous blood circuit 1b and therefore detects the hematocrit value of the blood purified by the dialyzer 2 and returning to the patient. When a local concentration variation is imparted by opening the electromagnetic valve 14, as illustrated in Fig. 4, a local concentration variation α having a unique peak P1 is detected by the second detector 5b. If such blood reaches the arterial blood circuit 1a again and undergoes recirculation, a local concentration variation β remaining in the recirculating blood and having a unique peak P2 is detected by the first detector 5a.

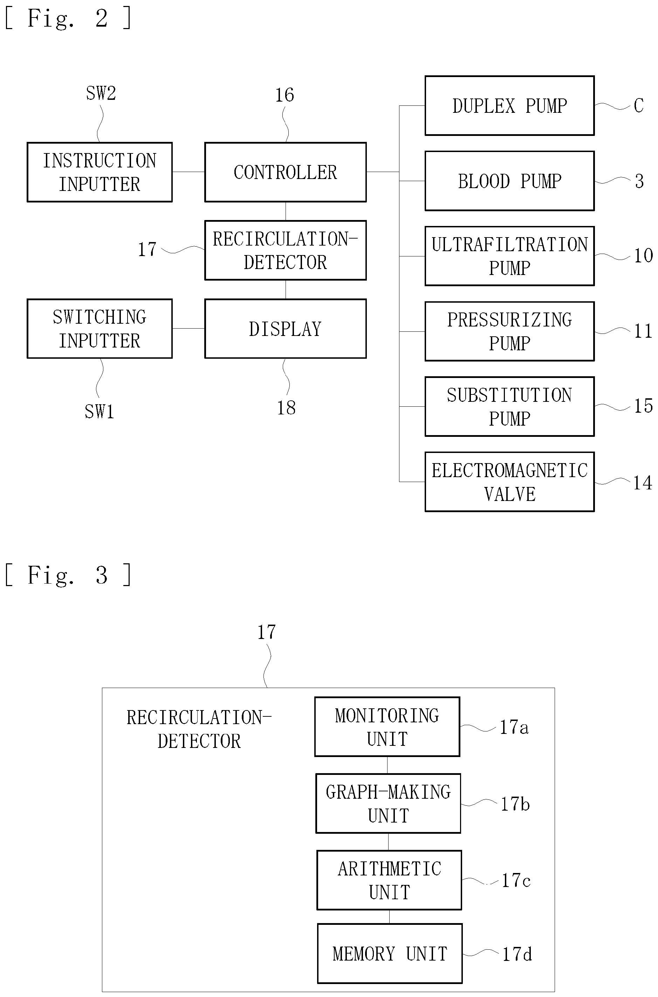

The controller 16 is, for example, a microcomputer or the like. As illustrated in Fig. 2, the controller 16 is electrically connected to and capable of controlling the following pumps and electromagnetic valves. The pumps include the duplex pump C configured to introduce and deliver dialysate into and from the dialyzer 2 (blood purification unit), the blood pump 3 provided to the blood circuit 1, the ultrafiltration pump 10 configured to remove water from the blood extracorporeally circulating through the blood circuit 1, the pressurizing pump 11, the substitution pump 15 configured to introduce the substitution fluid (dialysate) into the blood circuit, and a liquid-drug-infusing pump (not illustrated) configured to infuse a liquid drug such as heparin into the blood circuit 1. The electromagnetic valves include the electromagnetic valve 14 capable of imparting a local concentration variation having a unique peak to the fluctuating blood concentration.

As illustrated in Fig. 2, the controller 16 according to the present embodiment is connected to an instruction inputter SW2. An operator can make an input for instruction by operating the instruction inputter SW2. The instruction inputter SW2 only needs to allow the operator to make an input with any timing and may be, for example, a switch part displayed on a touch panel (including the display 18) or a mechanical switch.

The first detector 5a and the second detector 5b according to the present embodiment are electrically connected to the recirculation-detector 17 provided in the dialysis device 6. The recirculation-detector 17 is electrically connected to the display 18, which is constituted by a touch panel. The display 18 is configured to display, in the form of a graph, the local concentration variations α and β detected by the first detector 5a and the second detector 5b. In addition to displaying the graph, the display 18 is capable of displaying numerical values and/or the like representing the rate of recirculating blood and/or the like calculated by the recirculation-detector 17.

As illustrated in Fig. 2, the display 18 according to the present embodiment is connected to a switching inputter SW1. The operator can make an input for switching by operating the switching inputter SW1. The switching inputter SW1 only needs to allow the operator to make an input with any timing and may be, for example, a switch part displayed on a touch panel (including the display 18) or a mechanical switch.

The recirculation-detector 17 is, for example, a microcomputer or the like. The recirculation-detector 17 is capable of detecting any recirculating blood using the local concentration variation β detected by the first detector 5a and the local concentration variation α detected by the second detector 5b, and calculating the rate of recirculating blood (recirculation rate). The recirculating blood is blood reintroduced into the arterial blood circuit 1a after being returned to the patient from the venous blood circuit 1b. As illustrated in Fig. 3, the recirculation-detector 17 according to the present embodiment includes a monitoring unit 17a, a graph-making unit 17b, an arithmetic unit 17c, and a memory unit 17d.

The monitoring unit 17a is configured to monitor the fluctuating blood concentrations detected by the first detector 5a and the second detector 5b on a time-course basis during blood purification treatment. The graph-making unit 17b is configured to make a graph illustrating the local concentration variations α and β from the fluctuating blood concentrations monitored on a time-course basis by the monitoring unit 17a. The arithmetic unit 17c is configured to calculate the proportion of recirculating blood (recirculation rate) using the graph made by the graph-making unit 17b. The fluctuating blood concentrations monitored by the monitoring unit 17a, the graph made by the graph-making unit 17b, and the proportion of recirculating blood (recirculation rate) calculated by the arithmetic unit 17c are stored in the memory unit 17d.

Specifically, the recirculation-detector 17 acquires fluctuations in the hematocrit values (fluctuating concentrations) detected by the first detector 5a and the second detector 5b from the graph having a horizontal axis representing time course (integrated flow rate or time) and a vertical axis representing blood concentration (hematocrit value) as illustrated in Fig. 4, and calculates an area S1 of the local concentration variation α having the unique peak P1 and an area S2 of the local concentration variation β having the unique peak P2 by a mathematical method such as integration. The proportion of recirculating blood (recirculation rate) Rrec is calculated by the following mathematical expression.$Rrec \left(%\right) = S 2 / S 1 \times 100$

The proportion of recirculating blood (recirculation rate) Rrec calculated as above is displayed on the display 18 so as to be visually recognizable by a medical worker such as a doctor. If no blood recirculation occurs, S2 in the above expression becomes 0. Accordingly, the value displayed as the proportion of recirculating blood is 0 (%). The display 18 according to the present embodiment is capable of displaying not only the recirculation rate but also the local concentration variations detected by the first detector 5a and the second detector 5b in the form of a graph as illustrated in Fig. 4. Specifically, the display 18 is capable of displaying a graph illustrating curves representing the local concentration variation α having the unique peak P1 and the local concentration variation β having the unique peak P2.

The recirculation-detector 17 is configured to make the graph illustrating the local concentration variations α and β from the blood concentrations detected by the first detector 5a and the second detector 5b, with the horizontal axis representing time course (integrated flow rate or time) and the vertical axis representing blood concentration (hematocrit value) as illustrated in Fig. 4. Furthermore, the recirculation-detector 17 is configured to monitor the fluctuating blood concentrations every predetermined period (a period of 0.1 s, for example). Such a configuration enables the detection of the start point R1 and the end point R2 of the local concentration variation α having the unique peak P1 and the start point W1 and the end point W2 of the local concentration variation β having the unique peak P2, and thus enables accurate calculation of the area S1 of the local concentration variation α and the area S2 of the local concentration variation β.

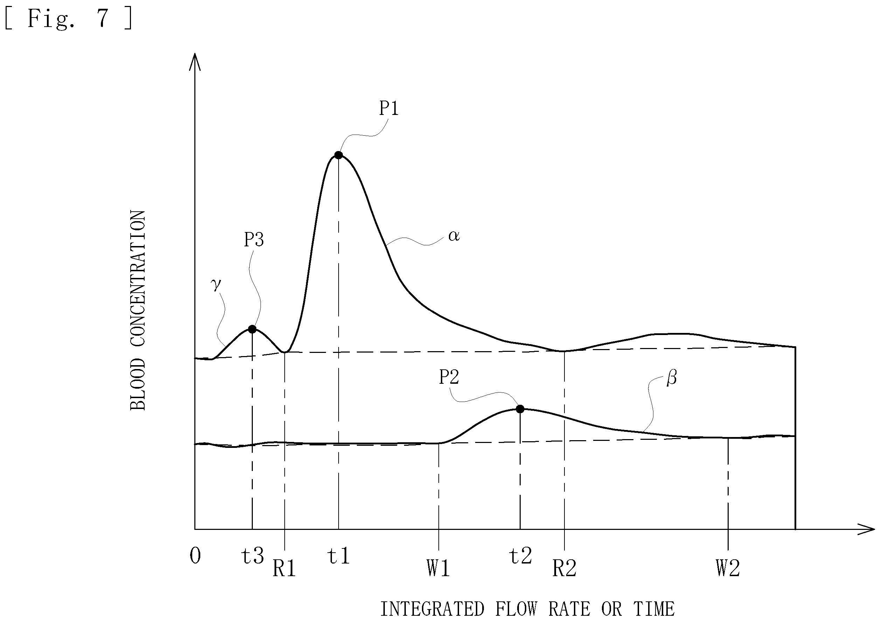

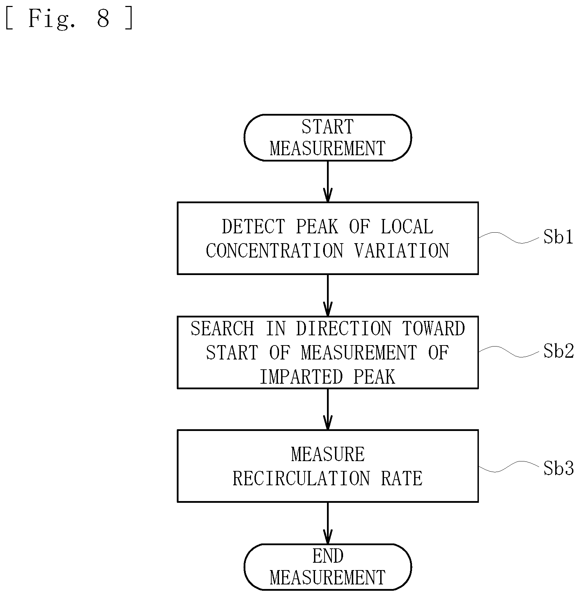

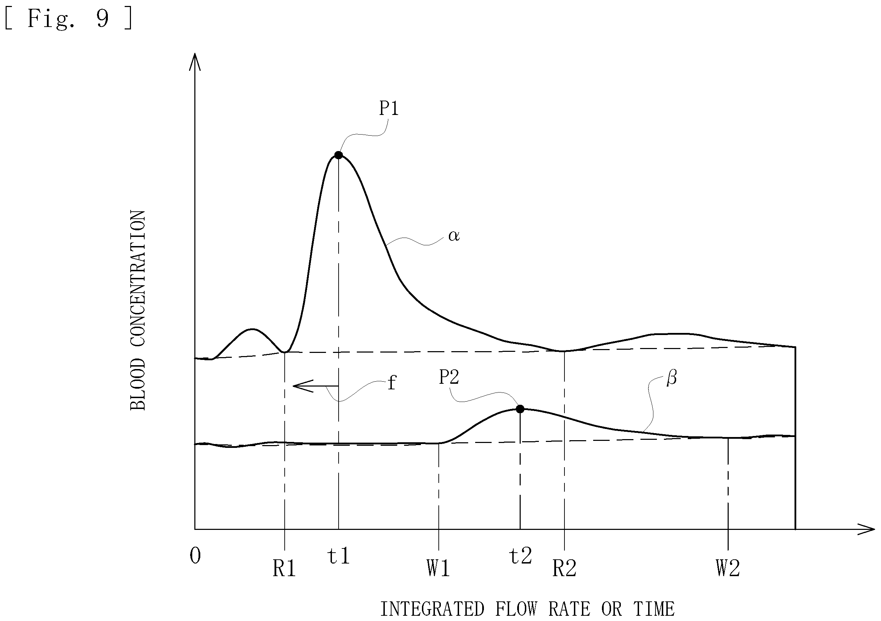

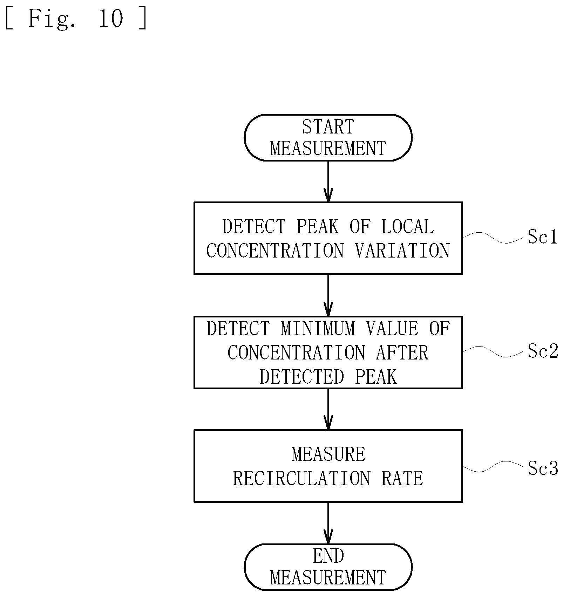

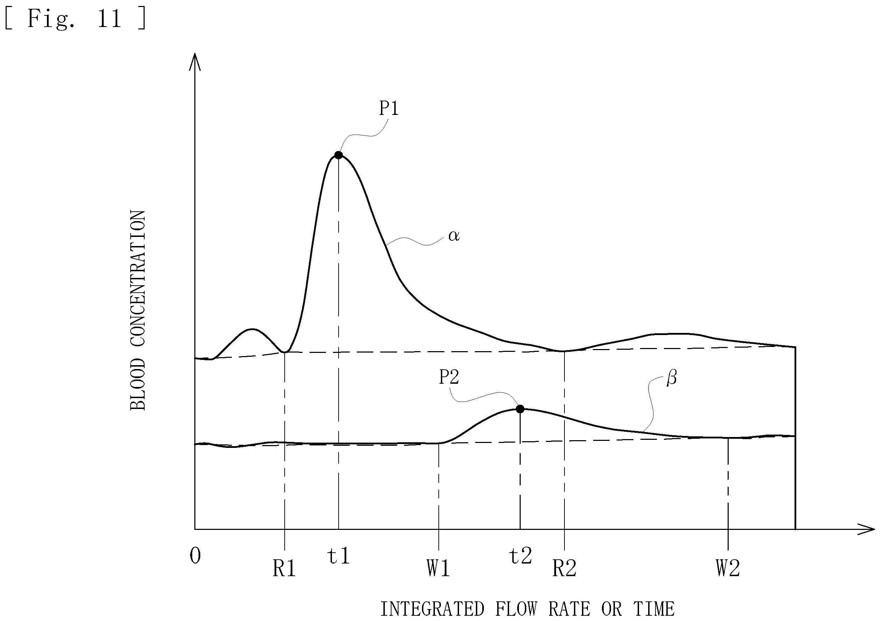

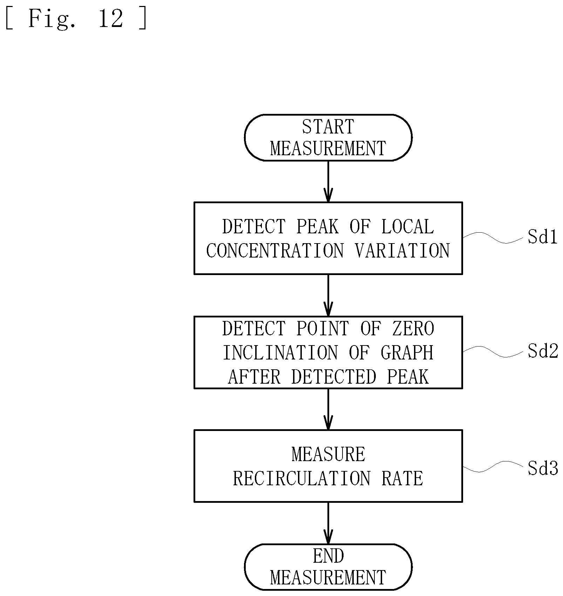

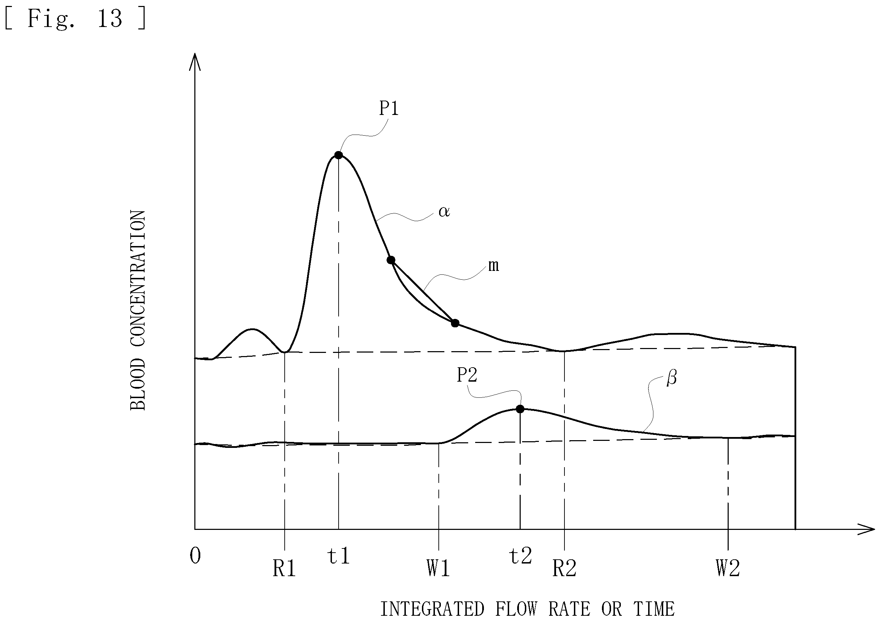

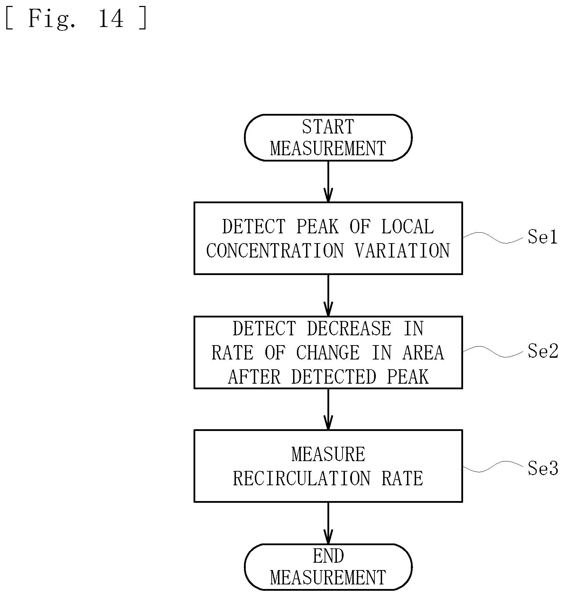

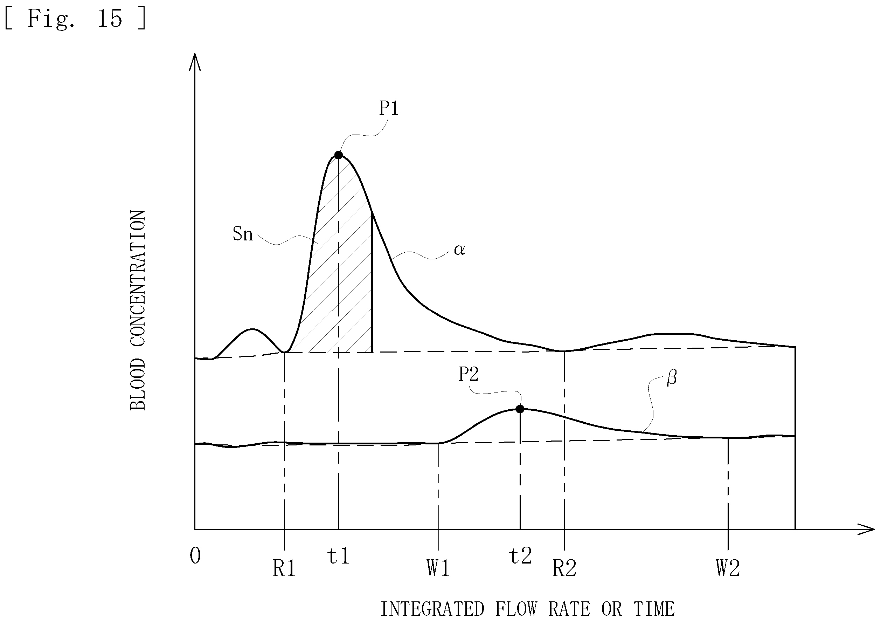

In the blood purification apparatus according to the present embodiment, the display 18 is configured to display the local concentration variations (α and β) detected by the first detector 5a and the second detector 5b in the form of a graph as illustrated in Fig. 7, and the recirculation-detector 17 is configured to calculate the area S1 and the area S2 by identifying the start points (R1 and W1) or the end points (R2 and W2) of the local concentration variations (α and β) detected by the first detector 5a and the second detector 5b.

The start points (R1 and W1) or the end points (R2 and W2) of the local concentration variations (α and β) displayed in the form of a graph on the display 18 are provided with indicators (D1 to D4) that indicate the start points (R1 and W1) or the end points (R2 and W2). As illustrated in Fig. 7, the indicators (D1 to D4) are bold-line representations, for example. The operator such as a medical worker is allowed to touch and move, as desired, any of the indicators (D1 to D4) with his/her finger (specifically, to slide it either leftward or rightward with the finger touching it). Thus, the positions of the indicators (D1 to D4) are each shiftable leftward and rightward.

The indicators (D1 to D4) are each not limited to a bold-line representation and may each be a specific figure, pattern, character, or the like, as long as the indicators (D1 to D4) can indicate the start points (R1 and W1) or the end points (R2 and W2). In the present embodiment, the display 18 is a touch panel, and the indicators (D1 to D4) are displayed on the screen of the display 18 and are each slidable with a finger. Alternatively, the movement of any of the indicators (D1 to D4) to a desired position may be implemented through an operation of another device included in the blood purification apparatus (such as an operation key that is physically operable to move the position of representation leftward and rightward).

The changing operation unit 19 is, for example, a microcomputer or the like and is configured to change, as desired, the start points (R1 and W1) or the end points (R2 and W2) identified by the recirculation-detector 17. The start points (R1 and W1) or the end points (R2 and W2) are changeable in response to the operator sliding the indicators (D1 to D4) as desired. The recirculation-detector 17 is configured to recalculate the rate of recirculating blood using the local concentration variations (α and β) having the start points (R1 and W1) or the end points (R2 and W2) changed by the changing operation unit 19 as desired.

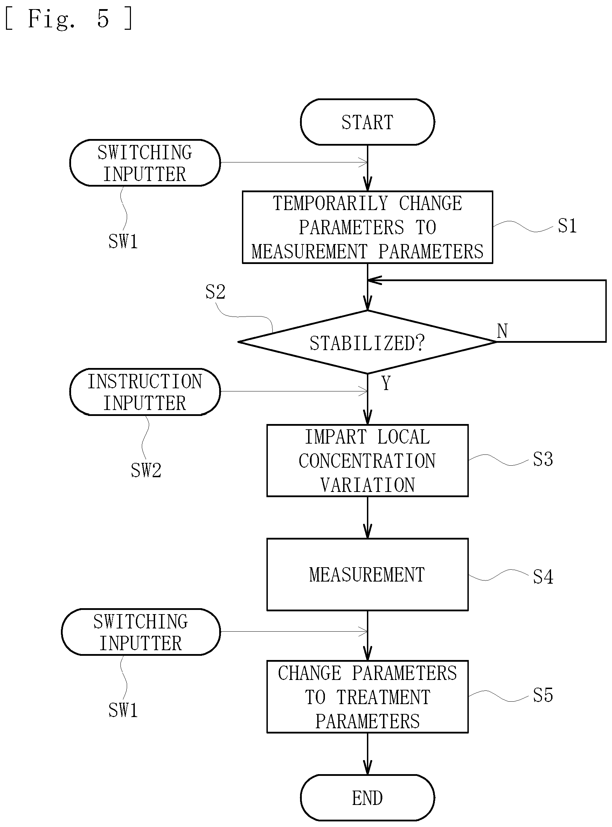

In the present embodiment, the process in which the recirculation-detector 17 detects any recirculating blood and calculates the rate of recirculating blood is performed in a measurement process, which is different from a treatment process of performing blood purification treatment. In the measurement process, the controller 16 performs a control operation after changing parameters intended for the activation of the pumps to be controlled (the duplex pump C, the blood pump 3, the ultrafiltration pump 10, the pressurizing pump 11, the substitution pump 15, the liquid-drug-infusing pump, and so forth) to measurement parameters, which are different from those for the treatment process of performing blood purification treatment. The measurement process will now be described with reference to a flow chart illustrated in Fig. 5.

First, the measurement process is started on condition that an input operation is performed on the switching inputter SW1 by an operator. In response to this, in step S1, the controller 16 temporarily changes the parameters of the pumps to be controlled to the measurement parameters, and activates the pumps in accordance with the measurement parameters. Then, in step S2, the controller 16 waits until the blood concentrations detected by the first detector 5a and the second detector 5b are stabilized. The measurement parameters are parameters that cause the pumps (pumps to be controlled) to operate at a lower speed than in the treatment process or to stop operating. After the blood concentrations detected by the first detector 5a and the second detector 5b are stabilized, the process proceeds to step S3 on condition that an input operation is performed on the instruction inputter SW2 by the operator. In step S3, the atmosphere release line 13 is opened by operating the electromagnetic valve 14, whereby a local concentration variation that forms unique peaks P1 and P2 is imparted.

Subsequently, the process proceeds to step S4, where the recirculation-detector 17 detects any recirculating blood using a local concentration variation α detected by the second detector 5b and a local concentration variation β detected by the first detector 5a, and calculates the rate of recirculating blood (recirculation rate). The rate of recirculating blood (recirculation rate) thus calculated is displayed on the display 18 so as to prompt a medical worker such as a doctor to take an appropriate action. After any recirculating blood is detected and the rate of recirculating blood (recirculation rate) is calculated, the process proceeds to step S5 on condition that an input operation is performed on the switching inputter SW1 by the operator. In step S5, the controller 16 changes the measurement parameters to the treatment parameters. Thus, the measurement process ends, and blood purification treatment is allowed to be performed in accordance with the treatment parameters.

To summarize, in the measurement process, the recirculation-detector 17 detects any recirculating blood and calculates the rate of recirculating blood (recirculation rate), and the display 18 displays, in the form of a graph, the local concentration variations (α and β) detected by the first detector 5a and the second detector 5b. The graph thus displayed is provided with the indicators (D1 to D4) at the start points (R1 and W1) and the end points (R2 and W2) of the local concentration variations (α and β).

When any of the indicators (D1 to D4) that is desired to change is slid leftward or rightward with a finger, a corresponding one of the start points (R1 and W1) or the end points (R2 and W2) is changed by the changing operation unit 19 as desired. Using the start points (R1 and W1) or the end points (R2 and W2) thus changed, the recirculation-detector 17 detects any recirculating blood and calculates the rate of recirculating blood (recirculation rate). The rates of recirculating blood (recirculation rates) calculated by the recirculation-detector 17 before the change and after the change are individually displayable on the display 18 so that the medical worker such as a doctor can conduct a simulation.

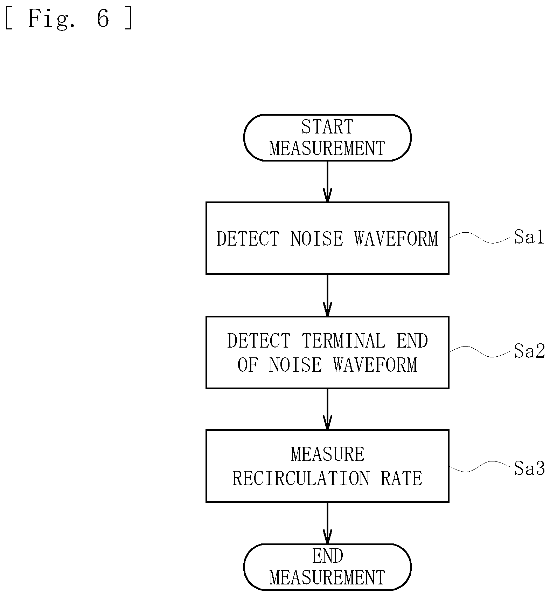

Now, a control process relevant to a changing operation that is performed by the changing operation unit 19 will be described with reference to a flow chart illustrated in Fig. 6.

In step S1a included in the measurement step S4, the result of the measurement is displayed (the indicators (D1 to D4) are displayed at the start points (R1 and W1) and the end points (R2 and W2) of the local concentration variations (α and β)). Then, the process proceeds to step S2a on condition that an input operation is performed on the instruction inputter SW2. In step S2a, whether any changing operation is performed by the changing operation unit 19 is determined.

If it is determined in step S2a that a changing operation is performed by the changing operation unit 19, the process proceeds to step S3a, where the start points (R1 and W1) or the end points (R2 and W2) are changed as desired in accordance with the changing operation performed by the changing operation unit 19. Subsequently, the process proceeds to step S4a, where the recirculation-detector 17 detects any recirculating blood using the start points (R1 and W1) or the end points (R2 and W2) thus changed, and calculates the rate of recirculating blood (recirculation rate).

If it is determined in step S2a that no changing operation is performed by the changing operation unit 19, the process proceeds to step S4a, skipping step S3a. That is, the recirculation-detector 17 detects any recirculating blood using the start points (R1 and W1) or the end points (R2 and W2), and calculates the rate of recirculating blood (recirculation rate). After the rate of recirculation blood (recirculation rate) is calculated in step S4a, the measurement process ends on condition that an input operation is performed on the switching inputter SW1 by the operator.

According to the present embodiment, the recirculation-detector 17 is configured to identify the start points (R1 and W1) or the end points (R2 and W2) of the local concentration variations (α and β) detected by the first detector 5a and the second detector 5b. Furthermore, the blood purification apparatus includes the changing operation unit 19 configured to change the identified start points (R1 and W1) and end points (R2 and W2) as desired. Such a configuration enables the operator such as a medical worker to determine the start points (R1 and W1) or the end points (R2 and W2) of the local concentration variations (α and β) as desired, and to simulate the calculation of the rate of recirculating blood.

While an embodiment has been described above, the present invention is not limited thereto. For example, the characteristic-variation-imparter is not limited to the one constituted by the atmosphere release line 13 and the electromagnetic valve 14 and may be any other means, as long as the characteristic-variation-imparter is capable of imparting a local concentration variation having a unique peak. The characteristic-variation-imparter according to the above embodiment is configured to concentrate the blood through ultrafiltration so as to make a local variation in blood concentration. Alternatively, for example, the local variation in blood concentration may be made by introducing a fluid such as a substitution fluid (dialysate) into the blood circuit so as to dilute the blood.

The first detector 5a and the second detector 5b may each be any device (such as a sensor configured to detect the concentration of hemoglobin, or a sensor configured to detect the concentration of protein or the like) other than a hematocrit sensor, as long as the device is capable of detecting fluctuating blood concentration having unique peaks. The first detector 5a and the second detector 5b may each be provided at any position of a corresponding one of the arterial blood circuit 1a and the venous blood circuit 1b.

While the fluctuating characteristic of blood according to the present embodiment is the fluctuating concentration of blood, the fluctuating characteristic of blood may be another fluctuating characteristic such as blood temperature. For example, ultrasonic waves may be used as follows: with a marker (such as physiological saline or microbubbles) infused into the blood, the curve of dilution may be measured with an ultrasonic probe to observe changes in bloodstream in the blood circuit on a real-time basis, whereby the recirculation rate may be quantified.

As another example, with a pigment infused into the arterial blood circuit, the light absorbance in the venous blood circuit may be measured to evaluate the occurrence of recirculation. As yet another example, with cooled physiological saline infused into the blood circuit, changes in blood temperature may be measured with temperature sensors provided to the arterial blood circuit and the venous blood circuit, whereby the recirculation rate may be calculated from the rate of change in the temperature. As yet another example, electrical impedance may be used as follows: changes in the electrical impedance of the blood in the blood circuit may be measured to analyze changes in the flow of blood, and a change in the impedance that is attributed to recirculation may be calculated using the electric conductivity of the physiological saline. As yet another example, optics may be used as follows: changes in the oxygen saturation of the blood or hemoglobin concentration may be measured by infrared spectroscopy (NIRS), and data for the arterial blood circuit and data for the venous blood circuit may be compared to evaluate the influence of recirculation.

According to a first aspect of the present invention, there is provided a blood purification apparatus configured to purify a patient's blood by causing the blood to extracorporeally circulate through a blood circuit 1 including an arterial blood circuit 1a and a venous blood circuit 1b. The blood purification apparatus includes a characteristic-variation-imparter (an atmosphere release line 13 and an electromagnetic valve 14) capable of imparting a local concentration variation to a fluctuating concentration of the blood extracorporeally circulating through the blood circuit 1, the local concentration variation having a unique peak; a first detector 5a and a second detector 5b provided to the arterial blood circuit 1a and the venous blood circuit 1b, respectively, and each being configured to detect the local concentration variation α and β imparted by the characteristic-variation-imparter; and a recirculation-detector 17 configured to detect any recirculating blood using the local concentration variation β detected by the first detector 5a and the local concentration variation α detected by the second detector 5b, and to calculate a rate of the recirculating blood, the recirculating blood being blood reintroduced into the arterial blood circuit 1a after being returned to the patient from the venous blood circuit 1b. The recirculation-detector 17 is configured to identify start points (R1 and W1) or end points (R2 and W2) of the local concentration variations (α and β) detected by the first detector 5a and the second detector 5b. The blood purification apparatus further includes a changing operation unit 19 configured to change the identified start points (R1 and W1) or end points (R2 and W2) as desired. Such a configuration enables an operator such as a medical worker to determine the start points (R1 and W1) or the end points (R2 and W2) of the local concentration variations (α and β) as desired, and to simulate the calculation of the rate of recirculating blood.

According to a second aspect of the present invention, in the first aspect, the recirculation-detector 17 is configured to calculate the rate of the recirculating blood using the local concentration variations (α and β) having the start points (R1 and W1) or the end points (R2 and W2) changed by the changing operation unit 19 as desired. Such a configuration enables the recalculation and update of the rate of recirculating blood using the local concentration variations (α and β) having the start points (R1 and W1) or the end points (R2 and W2) changed by the changing operation unit 19 as desired.

According to a third aspect of the present invention, the blood purification apparatus according to the first aspect further includes a display 18 configured to display the local concentration variations (α and β) detected by the first detector 5a and the second detector 5b in a form of a graph. The graph displayed by the display 18 is provided with indicators (D1 to D4) that indicate the start points (R1 and W1) or the end points (R2 and W2) of the local concentration variations (α and β). The changing operation unit 19 is configured to change the start points (R1 and W1) or the end points (R2 and W2) in response to an operator operating the indicators (D1 to D4) as desired. Such a configuration facilitates the operation of changing the start points (R1 and W1) or the end points (R2 and W2).

According to a fourth aspect of the present invention, in the third aspect, the changing operation unit 19 changes the start points (R1 and W1) or the end points (R2 and W2) in response to the indicators (D1 to D4) being moved on the display 18. Such a configuration enables intuitive operation of changing the start points (R1 and W1) or the end points (R2 and W2).

According to a fifth aspect of the present invention, in the first aspect, the characteristic of the blood is blood concentration. That is, the rate of recirculating blood can be calculated by using fluctuating blood concentration in detecting the recirculating blood.

### Industrial Applicability

The present invention is also applicable to any blood purification apparatus having a different appearance, additional functions, or the like within the spirit of the present invention.

### Reference Signs List

1 blood circuit
1a arterial blood circuit
1b venous blood circuit
2 dialyzer (blood purification unit)
3 blood pump
4a, 4b air-trap chamber
5a first detector
5b second detector
6 dialysis device
7 dialysate introduction line
8 dialysate drain line
9 bypass line
10 ultrafiltration pump
11 pressurizing pump
12 bubble isolation chamber
13 atmosphere release line
14 electromagnetic valve
15 substitution pump
16 controller
17 recirculation-detector
18 display
19 changing operation unit
C duplex pump
La supply line
La1 pre-substitution supply line
La2 post-substitution supply line
D1 to D4 indicator
SW1 switching inputter
SW2 instruction inputter

## Claims

1. A blood purification apparatus configured to purify a patient's blood by causing the blood to extracorporeally circulate through a blood circuit including an arterial blood circuit and a venous blood circuit, the blood purification apparatus comprising:
a characteristic-variation-imparter capable of imparting a local characteristic variation to a fluctuating characteristic of the blood extracorporeally circulating through the blood circuit, the local characteristic variation having a unique peak;
a first detector and a second detector provided to the arterial blood circuit and the venous blood circuit, respectively, and each being configured to detect the local characteristic variation imparted by the characteristic-variation-imparter; and
a recirculation-detector configured to detect any recirculating blood using the local characteristic variation detected by the first detector and the local characteristic variation detected by the second detector, and to calculate a rate of the recirculating blood, the recirculating blood being blood reintroduced into the arterial blood circuit after being returned to the patient from the venous blood circuit,
the recirculation-detector being configured to identify start points or end points of the local characteristic variations detected by the first detector and the second detector, the blood purification apparatus further comprising a changing operation unit configured to change the identified start points or end points as desired.

2. The blood purification apparatus according to claim 1, wherein the recirculation-detector is configured to calculate the rate of the recirculating blood using the local characteristic variations having the start points and the end points changed by the changing operation unit as desired.

3. The blood purification apparatus according to claim 1, further comprising a display configured to display the local characteristic variations detected by the first detector and the second detector in a form of a graph, wherein the graph displayed by the display is provided with indicators that indicate the start points or the end points of the local characteristic variations, and wherein the changing operation unit is configured to change the start points or the end points in response to an operator operating the indicators as desired.

4. The blood purification apparatus according to claim 3, wherein the changing operation unit changes the start points or the end points in response to the indicators being moved on the display.

5. The blood purification apparatus according to claim 1, wherein the characteristic of the blood is blood concentration.
